Europäisches Patentamt

European Patent Office

Office européen des brevets

(11) Publication number: **0 510 238 A1**

## EUROPEAN PATENT APPLICATION

(21) Application number: **91106862.5**

(22) Date of filing: **26.04.91**

(51) Int. Cl.5: **C07C 31/20**, C07C 29/00, B01J 27/045

(43) Date of publication of application:
**28.10.92 Bulletin 92/44**

(84) Designated Contracting States:
**AT BE CH DE DK ES FR GB GR IT LI LU NL SE**

(71) Applicant: **MENON S.r.l.**
**Via XIII Giugno, 8**
**I-48100 Ravenna(IT)**

(72) Inventor: **Casale, Bruno**
**Strada Rondini, 14**
**I-28062 Cameri (Novara)(IT)**
Inventor: **Marini, Luigi**
**Via Matteotti, 15**
**I-28040 Varallo Pombia (Novara)(IT)**

(74) Representative: **Rambelli, Paolo et al**
**Jacobacci-Casetta & Perani S.p.A. Via Alfieri 17**
**I-10121 Torino(IT)**

(54) Process for producing lower poliols and mixtures thereof.

(57) A method of producing lower polyols and mixtures thereof comprising the hydrogenation and hydrogenolysis under pressure of higher polyols is carried out in the presence of a platinum-based catalyst supported on an inert substrate and modified by sulphides in order to increase its selectivity towards the production of glycols.

EP 0 510 238 A1

The present invention relates to the production of lower polyols, particularly glycols and mixtures thereof, by a method comprising the catalytic hydrogenation and hydrogenolysis of higher polyols.

In the present description, the term higher polyols means products such as sorbitol, mannitol and xylitol which result from the catalytic hydrogenation of carbohydrates (particularly glucose).

The term lower polyols means polyalcohols having a maximum of six carbon atoms and a maximum of three hydroxyl groups, particularly ethylene glycol, propylene glycol and glycerine.

Known methods of producing lower polyols include single-step hydrogenation methods which use higher polyols as the starting products and two-step hydrogenation methods which use carbohydrates as the starting products.

With reference to the former methods, U.S. patent 2,004,135 describes a method of hydrogenating polyols, particularly sorbitol, in the presence of a nickel catalyst and a weakly alkaline promoter at a temperature of the order of 200-300°C and a pressure of the order of 210-280 atm.

In order to increase the glycerol yield, U.S. patent 2,965,679 proposes a method for the catalytic hydrogenation of polyols in alkaline aqueous solution under high pressures of the order of 500-1000 atm.

U.S. patent 4,362,332 describes the continuous catalytic hydrogenation of a solution of alditols on a static bed with a nickel catalyst and a basic promoter and with regeneration of the catalyst.

All the methods of the patents mentioned above have the particular object of increasing the glycerol yield; in order to increase the yield of glycols from sorbitol, U.S. patent 2,852,570 proposes the use of a catalyst containing nickel in combination with copper and/or cobalt with a hydrogen pressure of from 100 to 200 atm and a temperature of from 190 to 220°C.

With reference to the methods which use carbohydrates and, in particular, mono- and disaccharides as the starting products, U.S. patent 2,868,847 proposes the use of a ruthenium catalyst on an inert substrate for the carbohydrate-conversion step.

U.S. patent 4,380,678 describes a multi-step method of producing glycerine and other polyols in which a highly-active nickel catalyst is used both in the first step in which aldoses, particularly glucose, are hydrogenated to produce sorbitol, and in the second step for the hydrogenolysis of sorbitol.

U.S. patent 4,476,332 describes a two-step method in which a ruthenium catalyst modified with sulphides is used in the sorbitol-hydrogenolysis step; it is necessary to modify the ruthenium with sulphides in order to limit the hydrogenolysis reaction which would lead to the development of methane.

The method of the present invention aims in particular to achieve high selectivity towards the production of ethylene glycol and propylene glycol in the step for the catalytic hydrogenation and hydrogenolysis of higher polyols.

A further object of the invention is to provide a method for the continuous hydrogenation and hydrogenolysis of higher polyols to produce lower polyols with high yields. A further object is to provide a method which produces a mixture of glycols which, by virtue of its high 1,2 propylene glycol and ethylene glycol contents, its relatively low glycerol content, and the substantial absence of unconverted higher polyols, can be used as it is as an antifreeze mixture, particularly for motor-vehicle cooling systems.

These and further objects are achieved by a method of producing lower polyols and mixtures thereof comprising the hydrogenation and hydrogenolysis under pressure of higher polyols in the presence of a catalyst, characterised in that the catalyst is a platinum-based catalyst on an inert substrate and is modified by sulphides.

The platinum catalyst used may be a commercial catalyst, preferably on carbon, with a platinum content of between 1 and 7% by weight. The catalyst may be modified beforehand or in situ by the addition of a compound which can yield sulphur to the platinum. For this purpose preferred sulphur compounds are sodium sulphide, thiosulphates, particularly sodium thiosulphate, and bisulphites.

Conveniently, the reaction is carried out in the presence of a sufficient quantity of a basic promoter to bring the pH within the basic range from 8 to 13 and preferably between 9 and 11. The preferred basic promoters are alkali-metal and alkaline-earth hydroxides, particularly sodium and calcium hydroxides, and salts with basic reactions such as sodium carbonate and quaternary ammonium salts.

The operating conditions when the reaction is effected in batches, require a temperature between 200 and 280°C, preferably between 220 and 240°C, a total pressure between 50 and 200 bars (5-20 MPa), preferably between 100 and 150 bars (10-15 MPa), and reaction times of between two and four hours.

To advantage, the reaction is carried out continuously in a tubular reactor at a space velocity (LHSV) between 0.2 and 3 h$^{-1}$ and pressures between 50 and 150 bars (5-15 MPa), preferably between 100 and 150 bars (10-15 MPa). In the continuous method, the temperature may be kept lower than in the batch reaction, and preferably between 220 and 230°C.

The higher polyol or mixture of higher polyols is preferably supplied to the hydrogenation reactor in aqueous solution at a concentration of from 20 to 40% by weight. Particularly for the production of a mixture of lower polyols suitable for use as it is as an antifreeze mixture, possibly after partial dehydration, it is preferable to use sorbitol which is produced with high, almost unitary, yields by the hydrogenation of glucose. The sorbitol is supplied in an aqueous solution containing 20-40% by weight of sorbitol and including from 10 to 25 g/l of sodium hydroxide as the basic promoter and from 1 to 5 g/l of sodium sulphide.

According to another characteristic of the invention, the higher polyol is produced by a first step for the hydrogenation of carbohydrates, carried out at a weakly basic pH preferably between 7.5 and 8 and at a reaction temperature between 120 and 150ºC. The first step is also preferably carried out in aqueous solution in the presence of a basic promoter such as those described above which, in this step, is preferably constituted by a sufficient quantity of sodium hydroxide to keep the pH within the range indicated above. In this first step, the carbohydrates may be constituted by monosaccharides, disaccharides or polysaccharides such as starch or hydrolysed starch. The preferred supply, however, is constituted by a solution of glucose in water.

Further advantages and characteristics of the method according to the invention will become clear from the appended examples which are not intended to limit the scope of the present invention.

EXAMPLES 1-9

Tests on the catalytic hydrogenation and hydrogenolysis of sorbitol were carried out in a batch reactor with various quantities of a 5% platinum catalyst supported on carbon in powder form. In each test, 250 cm$^3$ of an aqueous solution of sorbitol (320g/l), calcium hydroxide (21.6 g/l) and sodium sulphide nonahydrate (one mole of sulphide for each mole of platinum) were loaded into a 500 cm$^3$ reactor with stirring. After the reactor had been pressurised with hydrogen at 130 bars, it was heated and kept at the desired temperature for two hours. After cooling, gas samples were taken from the reactor whilst the discharged liquid was first weighed and then filtered to remove the catalyst. After treatment with oxalic acid to precipitate the calcium ions, the liquid samples were analysed by high-pressure liquid chromatography (HPLC). The gas samples were analysed by gas chromatography. The results of the tests are given in Table 1 below in which:
- the quantity of catalyst used is expressed as the ratio r between the weight of sorbitol supplied to the reactor and the weight of metallic platinum introduced in the catalyst,
- the conversion is expressed as the ratio between the weight of sorbitol which reacted and the initial weight of sorbitol, and
- the selectivity towards each particular component is expressed as the ratio between the sorbitol converted into that component and the total sorbitol reacted.

The compounds for which selectivity values are given are glycerine, lactic acid, ethylene glycol, propylene glycol, the butanediols (as a mixture of 1,2 and 2,3 butanediol), the alcohols (as a mixture of methanol, ethanol, isopropanol and normal propanol) and the gases (mainly methane).

Table 1

| (Examples 1-9) Batch tests on the hydrocracking reaction | | | | | | | | | | |
|---|---|---|---|---|---|---|---|---|---|---|
| Example | Temp. (ºC) | r | conversion % | Selectivity % | | | | | | |
| | | | | Gly | Al | EG | PG | Bu | Alc | Gas |
| 1 | 250 | 160 | 99.6 | 2.4 | 10.3 | 15.2 | 47.2 | 6.3 | 9.5 | 0.2 |
| 2 | 250 | 222 | 98.2 | 6.8 | 12.6 | 16 | 44 | 6.6 | 8.6 | 0.2 |
| 3 | 250 | 400 | 95 | 7.9 | 13.5 | 16.1 | 42 | 7.4 | 12.8 | 0.05 |
| 4 | 250 | 320 | 96.3 | 7 | 12 | 15.6 | 42 | 5.9 | 7.4 | 0.1 |
| 5 | 250 | 800 | 85.5 | 8.2 | 14.7 | 15.6 | 39.1 | 6.8 | 10.1 | 0.02 |
| 6 | 200 | 200 | 78.3 | 14.2 | 15.7 | 14.6 | 39.6 | 4.8 | 3.6 | 0.08 |
| 7 | 225 | 200 | 90.3 | 11.9 | 13.2 | 15.0 | 41.1 | 4.3 | 7.1 | 0.05 |
| 8 | 225 | 400 | 82 | 11.6 | 11.1 | 15.6 | 41.1 | 3.7 | 6 | 0.02 |
| 9 | 200 | 400 | 71.3 | 13.5 | 15.4 | 14.7 | 38.2 | 6.2 | 8.1 | 0.02 |

in which:
Gly:    glycerine
AL:     lactic acid
EG:     ethylene glycol

PG:    1,2 propylene glycol
Bu:    butandiols
Alc:   alcohols

It can be seen from Table 1 that conversion can be improved by raising the temperature and the quantity of catalyst (low r values); the temperature and the ratio r slightly affect selectivity towards ethylene glycol and propylene glycol but have a greater effect on selectivity towards glycerol. Selectivity towards the butandiols is between 4 and 8% whilst for the alcohols, values between 6 and 13% were obtained with higher values at higher temperatures.

Other products (1,2,4 butantriol, erythritol, 1,2 pentandiol) which are not given in the table were also identified. When all the components identified in the reaction liquid were taken into consideration, the total material balance (based on elementary carbon) was found to have a variable error within the range between 3 and 10%.

The quantity of methane produced was very low in all the tests (maximum selectivity 0.2%) by virtue of the presence of sulphides in the supply to the reactor.

EXAMPLES 10-11

These examples relate to tests on the hydrocracking of sorbitol to produce glycols carried out continuously with the use of a platinum catalyst at various temperatures and at a space velocity (LHSV) of $1h^{-1}$. A commercial platinum catalyst with a percentage of 4.2% by weight of platinum supported on dry granular carbon was used.

A static-bed reactor was used, with the downward supply at the same flow-rate of two streams: hydrogen presaturated by the injection of water, and a solution of sorbitol also containing sodium hydroxide and sodium sulphide nonahydrate. A preliminary step took place in the reactor to mix the reagents before they entered the catalytic bed. The two streams were suitably preheated (care being taken to keep the temperature to which the sorbitol solution was preheated below 150° to prevent its thermal decomposition) so as to achieve the desired temperature after the streams were mixed.

As regards the operating conditions, the tests of Examples 10-11 were carried out at a total pressure of 150 bars with a hydrogen/sorbitol molar ratio of 9:1 at the input of the reactor. The concentration of sorbitol in the liquid entering the catalytic bed was 330 g/l, that of the sodium sulphide nonahydrate was 5 g/l and that of the sodium hydroxide was 25 g/l.

The gaseous phase and the liquid phase of the mixture output from the reactor were separated; samples of the gaseous phase were analysed by gas chromatography as in Examples 1-9 above. The liquid phase was also analysed by the same method as in the previous examples. The results are given in Table 2 below.

Table 2

| (Examples 10-11) Continuous tests on the hydrocracking of sorbitol | | | | | | | | |
|---|---|---|---|---|---|---|---|---|
| Example | Temp. (°C) | conversion % | Selectivity % | | | | | |
| | | | Gly | AL | EG | PG | Bu | Alc |
| 10 | 250 | 99.8 | 3 | 2.6 | 16 | 44 | 17 | 5 |
| 11 | 225 | 98 | 5 | 1.8 | 17 | 47 | 14.1 | 2.3 |

With a space velocity value (LHSV) of $1h^{-1}$ conversion remained high even at low temperatures (225°C). Selectivity was also very high and varied with temperature since higher temperatures enabled the hydrogenation of products such as glycerol, propylene glycol and butandiols to form lighter products (alcohols). The production of methane was again negligible (its selectivity is not given in the table) by virtue of the presence of sodium sulphide as a moderator of the catalyst.

A total yield of 63-68% can be achieved under the conditions given in the table, if ethylene glycol, propylene glycol and glycerine are considered to be desired products. The total selectivity is about 90%, the rest being due to lost and unidentified substances.

EXAMPLES 12-15

These examples relate to tests on the continuous hydrocracking of sorbitol with a platinum catalyst containing 3% by weight of platinum on carbon. The tests were carried out by the same method as Examples 10-11, at various temperatures and with various periods of time spent in the reactor (LHSV). The results are given in Table 3 below.

Table 3

| (Examples 12-15) | | | | | | | | | |
|---|---|---|---|---|---|---|---|---|---|
| Example | Temp. (°C) | LHSV (h$^{-1}$) | conversion % | Selectivity % | | | | | |
| | | | | Gly | AL | EG | PG | Bu | Alc |
| 12 | 250 | 0.667 | 100 | 1.4 | 3.5 | 13 | 38 | 18 | 10 |
| 13 | 225 | 0.667 | 99 | 3.5 | 1.7 | 18 | 49 | 13.4 | 2.6 |
| 14 | 250 | 1 | 99.9 | 2.4 | 2.6 | 17 | 46 | 15.3 | 5.1 |
| 15 | 225 | 1 | 97.5 | 4.9 | 1.8 | 20 | 50 | 13.3 | 2.3 |

As can be seen from an examination of Table 3, long periods of time spent in the reactor (lower LHSV values) do not substantially affect the hydrocracking reaction; only slightly improved conversion at 225°C (from 97.5 to 99) and reduced selectivity towards glycerol (from 4.9 to 3.5%) were noted.

It is clear from a comparison of Tables 2 and 3 that conversion and selectivity do not change appreciably even if the percentage of the metallic catalyst is reduced.

EXAMPLES 16-18

Tests were carried out by the method of Examples 10-15 indicated above with the use of 1% by weight of a supported platinum catalyst. The reaction temperature was kept at 235°C. The results are given in Table 4 below.

Table 4

| (Examples 16-18) | | | | | | | | |
|---|---|---|---|---|---|---|---|---|
| Example | LHSV (h$^{-1}$) | conversion % | Selectivity % | | | | | |
| | | | Gly | AL | EG | PG | Bu | Alc |
| 16 | 2.5 | 22.5 | 8.6 | 6.7 | 6 | 16 | 10 | 3 |
| 17 | 1.66 | 20 | 2 | 8 | 9 | 25 | 15 | 6 |
| 18 | 1.25 | 17 | 2 | 9.3 | 10 | 28 | 21 | 8.5 |

The value of 1% of platinum for the catalyst may thus be considered a limit value since both conversion and selectivity towards ethylene glycol and propylene glycol are considerably reduced.

Naturally, it is intended that, the principle of the invention remaining the same, the forms of embodiment of the method according to the invention may be varied widely with respect to those described and illustrated in the examples, without thereby departing from the scope of the present invention.

**Claims**

1. A method of producing lower polyols and mixtures thereof comprising the hydrogenation and hydrogenolysis under pressure of higher polyols in the presence of a catalyst, characterised in that the catalyst is a platinum-based catalyst on an inert substrate and is modified by sulphides.

2. A method according to Claim 1, characterised in that the reaction temperature is between 200 and 280°C and the total pressure is between 5 and 20 MPa.

3. A method according to Claim 1, characterised in that the reaction is carried out continuously on a static catalyst bed.

**4.** A method according to Claim 3, characterised in that the reaction temperature is between 220 and 230°C and the pressure is between 5 and 15 MPa.

**5.** A method according to Claim 3 or Claim 4, characterised in that the space velocity (LHSV) is between 0.2 and 3 h⁻¹.

**6.** A method according to any one of the preceding claims, characterised in that a percentage of between 1 and 7 by weight of the platinum catalyst is supported on carbon.

**7.** A method according to any one of the preceding claims, characterised in that the reaction is carried out in the presence of a basic promoter selected from the group consisting of alkali-metal and alkaline-earth hydroxides and salts with basic reactions.

**8.** A method according to any one of the preceding claims, characterised in that the ratio of sulphide ions to platinum is between 0.5 and 2 moles of sulphide ions per mole of platinum.

**9.** A method according to any one of the preceding claims, characterised in that the higher polyol is the product obtained by a first step in which the catalysed hydrogenation and hydrogenolysis of carbohydrates is carried out at a weakly basic pH.

**10.** A method according to Claim 9, characterised in that the pH in the first hydrogenation and hydrogenolysis step is between 7.5 and 8.

**11.** A method according to Claim 10, characterised in that the pH between 7.5 and 8 is achieved by the addition of an alkali-metal or alkaline-earth hydroxide.

**12.** A method for the hydrogenation and hydrogenolysis of carbohydrates, characterised in that it includes a first step in which a mixture of carbohydrates and hydrogen is contacted, under weakly basic conditions, with a platinum-based catalyst supported on carbon to produce higher polyols, and a second step in which the higher polyols thus produced are hydrogenated in contact with a platinum-based catalyst supported on carbon and modified with sulphides, to produce a mixture of lower polyols.

**13.** A method according to any one of the preceding claims, characterised in that the higher polyol is sorbitol.

**14.** A method according to any one of Claims 9, 10 and 11, characterised in that the carbohydrate is glucose.

**15.** The use of the mixture of lower polyols produced by the method according to any one of the preceding claims as an antifreeze mixture particularly for motor-vehicle cooling systems.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | US-A-4430253 (M. DUBECK ET AL.)<br>* column 3, line 4 - column 3, line 21 *<br>--- | 1, 2,<br>7-14 | C07C31/20<br>C07C29/00<br>B01J27/045 |
| Y | Fieser & Fieser: "Reagents for organic synthesis"<br>1967, John Wiley and sons, London GB<br>* page 892, line 22 - page 892, line 27 *<br>--- | 1, 2,<br>7-14 | |
| A | CH-A-342554 (INVENTA AG)<br>* claims *<br>--- | 1 | |
| A | EP-A-72629 (DU PONT)<br>* page 5, line 19 *<br>--- | 1 | |
| A | US-A-4496780 (B.J. ARENA)<br>* claim 1 *<br>----- | 1 | |

TECHNICAL FIELDS
SEARCHED (Int. Cl.5 )

C07C
B01J

The present search report has been drawn up for all claims

| Place of search | Date of completion of the search | Examiner |
|---|---|---|
| BERLIN | 19 DECEMBER 1991 | PROBERT C. |

EPO FORM 1503 03.82 (P0401)